# EUROPEAN PATENT APPLICATION

(11) **EP 3 103 460 A1**
(43) Date of publication of application: **14.12.2016**
(21) Application number: 15746220.1
(22) Date of filing: 06.02.2015
(51) Int. Cl.: A61K 33/38, A61K 33/18, A61K 47/12, A61K 47/32, A61P 17/00, A61P 31/04

(54) **BACTERICIDAL COMPOSITION FOR BACTERIAL SKIN DISEASE**

(30) Priority: 07.02.2014 JP 2014022223
(71) Applicant: NBC Meshtec, Inc., Hino-shi, Tokyo 191-0053 (JP)
(72) Inventor: FUKUI, Yoko, Hino-shi Tokyo 191-0053 (JP); MAZUYAMA, Eri, Hino-shi Tokyo 191-0053 (JP); FUJIMORI, Yoshie, Hino-shi Tokyo 191-0053 (JP); NAGAO, Tomokazu, Hino-shi Tokyo 191-0053 (JP); NAKAYAMA, Tsuruo, Hino-shi Tokyo 191-0053 (JP)
(74) Representative: Vossius & Partner Patentanwälte Rechtsanwälte mbB
(86) International application number: PCT/JP2015/000563
(87) International publication number: WO 2015/118885

(57) **Abstract**

[Problem] To provide a bactericidal composition for bacterial skin disease against which it is difficult for bacteria that cause bacterial skin disease to acquire drug resistance.

[Solution] A bactericidal composition for bacterial skin disease that is characterized by including silver iodide in the form of nanoparticles having a particle size of 1-100 nm, inclusive, iodide ions, a water-soluble polymer, and an organic acid having a carboxyl group, and/or a salt of said acid, wherein the molar ratio of silver to iodide ions in the silver iodide is 1:1-1:1000.

## Description

### Technical Field

The present invention relates to a bactericidal composition for bacterial skin disease containing silver iodide nanoparticles.

### Background Art

In Japan, about 20,000,000 dogs and cats are reared as pets a year. Due to the small dog boom in recent years and the absence of outdoor places for rearing pets, indoor dogs living with humans are increasing. However, pets living indoors are excessively shampooed by their owners who are annoyed by pet odors. Accordingly, skin barrier layers naturally possessed by pets are disrupted, and the pets are infected with bacteria, mold, and viruses which hide in the human's living environment. Thus, the real fact is that pets affected with various diseases are increasing.

Pets are affected with various diseases, and skin diseases make up 30% of the various diseases. There are various types of skin diseases caused by bacterial infection, fungal infection, allergy, parasites, and the like. Especially problematic is a bacterial skin disease called pyoderma in which abnormal proliferation of bacteria normally present in the skin or the like leads to a red rash associated with a strong itch. A treatment of these includes cleaning the skin of a pet with an antibacterial shampoo. When the disease is serious, for example, an oral medicine containing an antibiotic is commonly administered. It is noted that as an antibacterial compound which can be used for pyoderma, the compound disclosed in Patent Literature 1, for example, is proposed.

### Citation List

### Patent Literature

Patent Literature 1: JP2003-034637

### Summary of Invention

### Technical Problem

Many of the compositions for bacterial skin diseases having been proposed in the conventional cases include an antibiotic. This causes drug resistant bacteria to appear, making it difficult to cure the disease completely.

To address this concern, the present invention is to provide a bactericidal composition for bacterial skin disease to which bacteria responsible for bacterial skin diseases are unlikely to acquire drug resistance.

### Solution to Problem

That is, a first invention is a bactericidal composition for bacterial skin disease, including silver iodide in the form of nanoparticles having a particle size of 1 nm or more and 100 nm or less, iodide ions, a water-soluble polymer, and an organic acid having a carboxyl group and/or a salt thereof, wherein the molar ratio of silver in the silver iodide and the iodide ions is 1:1 to 1:1000.

A second invention is the bactericidal composition for bacterial skin disease according to the first invention, further including at least one selected from the group consisting of a surfactant, a humectant, a solvent, and an antioxidant.

A third invention is a bactericidal composition for bacterial skin disease, manufactured by:
setting the molar ratio of silver ions constituting a silver salt and iodide ions constituting an iodide such that silver iodide in the form of nanoparticles having a particle size of 1 nm or more and 100 nm or less is contained in the obtained composition, and the molar ratio of silver contained in the silver iodide and iodide ions existing as ions becomes 1:1 to 1:1000; and
mixing, in a solvent, the silver salt and the iodide in which the molar ratio of the silver ions and the iodide ions is set, a water-soluble polymer, and an organic acid having a carboxyl group and/or a salt thereof.

A fourth invention is a manufacturing method of a bactericidal composition for bacterial skin disease, including:
setting the molar ratio of silver ions constituting a silver salt and iodide ions constituting an iodide such that silver iodide in the form of nanoparticles having a particle size of 1 nm or more and 100 nm or less is contained in the obtained composition, and the molar ratio of silver contained in the silver iodide and iodide ions existing as ions is 1:1 to 1:1000; and
mixing, in a solvent, the silver salt and the iodide in which the molar ratio of the silver ions and the iodine ions is set, a water-soluble polymer, and an organic acid having a carboxyl group and/or a salt thereof.

### Advantageous Effects of Invention

According to the present invention, there can be provided a bactericidal composition for bacterial skin disease to which bacteria responsible for bacterial skin diseases are unlikely to acquire drug resistance.

### Brief Description of Drawings

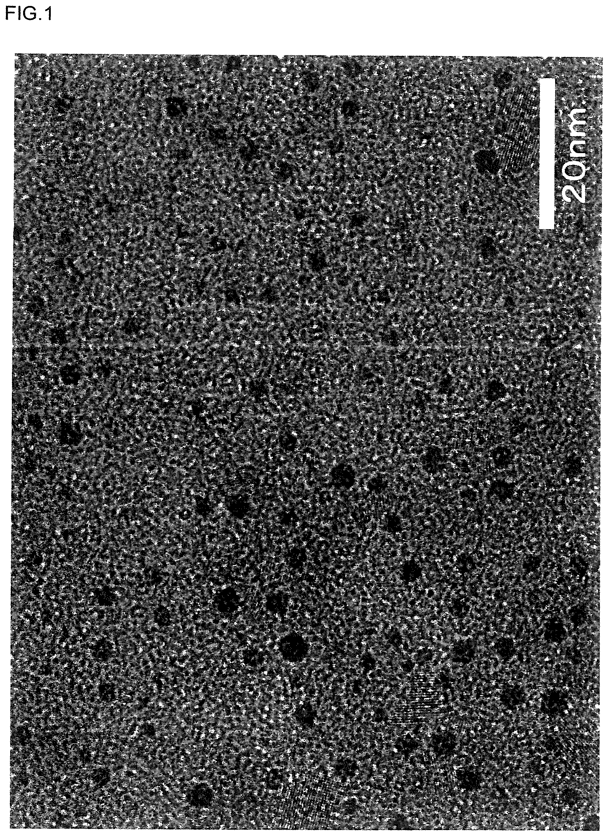

FIG 1 is an image of a bactericidal composition for bacterial skin disease obtained in Example 3 by a transmission electron microscope.

### Description of Embodiments

Hereinafter, an embodiment of a bactericidal composition for bacterial skin disease of the present invention will be described in detail. The bactericidal composition for bacterial skin disease of this embodiment includes nanoparticles made of silver iodide having a particle size of 1 nm or more and 100 nm or less (also referred to as silver iodide nanoparticles), iodide ions, a water-soluble polymer, and an organic acid having a carboxyl group (hereinafter, also simply referred to as an organic acid) and/or a salt thereof. Furthermore, in the bactericidal composition for bacterial skin disease of this embodiment, the molar ratio of silver in the silver iodide and the iodide ions (iodide ions that do not react with silver ions and exist as ions in the composition) is 1:1 to 1:1000.

The bactericidal composition for bacterial skin disease of this embodiment can be manufactured by, for example, mixing, in a solvent, a silver salt, an iodide, a water-soluble polymer, and an organic acid and/or a salt thereof. By mixing the silver salt and the iodide, the silver salt and the iodide react with each other to generate silver iodide. The ratio of each component is not particularly limited. However, the ratio of the silver ions constituting the silver salt and the iodide ions constituting the iodide is preferably set such that the molar ratio of the silver contained in the generated silver iodide nanoparticles and the iodide ions existing as ions in the composition becomes 1:1 to 1:1000. The mixing order or the like is not particularly limited. For example, the composition may be prepared by adding the iodide ions to a composition containing the already obtained silver iodide nanoparticles, the water-soluble polymer, and the organic acid and/or a salt thereof.

The silver iodide, which is contained in the bactericidal composition for bacterial skin disease of this embodiment, has been known to have bactericidal properties, and is utilized as various bactericidal compositions. In this embodiment, there can be used silver iodide having an extremely small particle size of 1 nm or more and 100 nm or less and having the form of nanoparticles. From the viewpoint of exerting bactericidal properties more efficiently even in a small amount, the particle size of the silver iodide nanoparticles is preferably as small as possible. On the other hand, when the particle size is less than 1 nm, stability of the silver iodide as a substance decreases. Therefore, the particle size of the silver iodide nanoparticles is more preferably a single nanosize of less than 10 nm and 1 nm or more. It is noted that the particle size can be controlled by, for example, adjusting the ratio of the silver ions contained in the silver salt and the iodide ions contained in the iodide when the silver salt reacts with the iodide.

As described above, the silver iodide nanoparticles can be obtained by, for example, a reaction of the silver salt with the iodide.

Examples of the silver salt may include silver nitrate, silver nitrite, silver chlorate, silver perchlorate, silver acetate, and silver sulfate. Examples of the iodide may include potassium iodide. Any combination of these silver salts and iodides may be freely selected.

Regarding the ratio of the silver salt and the iodide in the preparation of the bactericidal composition for bacterial skin disease described above as an example, it is desirable that the iodide is stoichiometrically excess relative to the silver salt. Specifically, the molar ratio of the silver ions constituting the silver salt and the iodide ions constituting the iodide is preferably set such that the molar ratio of the silver in the silver iodide existing in the bactericidal composition for bacterial skin disease of this embodiment, and the iodide ions (iodide ions that do not react with silver ions and exist as ions in the composition) becomes 1:1 to 1:1000. This is particularly preferable in this embodiment, because the larger the difference of the molar ratio between the silver ions and the iodide ions is, the smaller the particle size of the obtained silver iodide nanoparticles is.

When the ratio of the iodide ions is less than 1:1, all the iodide ions react with the silver ions in the preparation of the composition. Accordingly, a later-described electric double layer is not to be formed around the silver iodide nanoparticles. As a result, the zeta potential of the silver iodide nanoparticles decreases, causing the particles to aggregate and settle down, or causing the particles to be conjugated with each other leading to an increasedparticle size and settle down. Accordingly, dispersion stability of the silver iodide nanoparticles decreases. On the other hand, when the ratio of the iodide ions exceeds 1:1000, the iodide ions become too much, resulting in formation of a complex with silver.

Here, the electric double layer formed around the silver iodide nanoparticles in this embodiment will be described. The iodide exists as the iodide ions in the solution, and reacts with the silver ions produced by dissolution of the silver salt in water, thereby to generate nanoparticles of silver iodide. The generated silver iodide nanoparticles adsorb excess iodide ions existing in the solution to be charged with a minus charge. The adsorbed iodide ions further adsorb counter ions of the excess iodide ions. Thus, an electric double layer is formed around the silver iodide nanoparticles. The silver iodide nanoparticles, around which the electric double layer is formed, have high zeta potential, thereby achieving dispersion stability.

In this embodiment, the silver iodide nanoparticles having achieved dispersion stability are protected by the water-soluble polymer as a dispersant. Furthermore, the organic acid and/or a salt thereof suppress aggregation of the silver iodide nanoparticles. This enables dispersion stability of the silver iodide nanoparticles to be extraordinarily enhanced. Therefore, aggregation of the silver iodide nanoparticles can be suppressed for an extended period even when the particle size is several nm.

The water-soluble polymer is not particularly limited, as long as it enhances dispersibility of the nanoparticles. Examples of the water-soluble polymer may include a water-soluble macromolecular dispersant such as hydroxypropyl cellulose, polyvinyl pyrrolidone, and polyvinyl alcohol. One or two or more thereof may be included in the bactericidal composition for bacterial skin disease of this embodiment. Although these water-soluble polymers have various molecular weights, the water-soluble polymer having a molecular weight of 500 or more is suitably used, because as the molecular weight is higher, the obtained nanoparticles have a smaller particle size.

The ratio of the water-soluble polymer is not particularly limited. However, 0.1 parts by mass or more relative to 100 parts by mass of the whole composition is preferable from the viewpoint of further enhancing dispersibility of the silver iodide nanoparticles. The upper limit of the ratio of the water-soluble polymer is not particularly limited. However, in consideration of contact efficiency between bacteria and the silver iodide, the water-soluble polymer is preferably contained in a ratio of 1 part by mass or less relative to 100 parts by mass of the whole composition.

The organic acid having a carboxyl group (-COOH) and/or a salt thereof are not particularly limited. Specific examples of the organic acid may suitably include oxycarboxylic acids such as citric acid, lactic acid, malic acid, tartaric acid, succinic acid, ascorbic acid, glycolic acid, and salts thereof. For example, one or two or more thereof may be contained in the bactericidal composition for bacterial skin disease of this embodiment. Among these, citric acid is more suitably used.

The ratio of the organic acid and/or a salt thereof is not particularly limited. However, 0.01 parts by mass or more relative to 100 parts by mass of the whole composition is preferable from the viewpoint of improving bactericidal properties. The upper limit of the ratio of the organic acid and/or a salt thereof is not particularly limited. However, for reasons such as suppression of the reaction of the organic acid with the iodide ions resulting in coloring, the organic acid and/or a salt thereof is preferably contained in a ratio of 5 parts by mass or less relative to 100 parts by mass of the whole composition.

When a dispersion liquid containing the silver iodide nanoparticles having dispersion stability is prepared in this embodiment, it is important to prevent aggregation of the silver iodide nanoparticles. Aggregation of the silver iodide nanoparticles is significantly influenced by zeta potential possessed by the particles. Therefore, the organic acid and/or a salt thereof are used to maintain the pH of the dispersion liquid on the acidic side for increasing the zeta potential of the silver iodide nanoparticles thereby enabling suppression of aggregation. The added amount of the organic acid and/or a salt thereof is preferably an amount that allows the pH of the aqueous solution to be adj usted at 2.0 to 6. 0, from the viewpoint of further suppressing aggregation of the silver iodide nanoparticles. Furthermore, how the supply of the silver ions, as a main component of the silver iodide nanoparticles, from the solution is controlled becomes important for controlling the particle size of the silver iodide nanoparticles. That is, how particle growth of the silver iodide nanoparticles by the silver ions is inhibited becomes important. Here the silver ions include those not contributing to the reaction during the synthesis of the nanoparticles and remaining in the composition and those eluted from the nanoparticles due to the time-dependent change. Since the organic acid and/or a salt thereof are easy to form a complex with the silver ions and to be stabilized, they can inhibit the above-described silver ions from being involved in the growth of the particles. An oxycarboxylic acid, particularly citric acid, is especially easy to form a complex with the silver ions and to be stabilized. Thus, the use of the oxycarboxylic acid (more preferably, a citric acid) as a pH adjuster is more suitable in order to synthesize the silver iodide nanoparticles having an extremely small particle size of several nm and having excellent dispersion stability.

In the bactericidal composition for bacterial skin disease of this embodiment, it is preferable that, for example: the ratio of the silver ions constituting the silver salt and the iodide ions constituting the iodide be set such that the molar ratio of the silver contained in the generated silver iodide nanoparticles and the iodide ions existing as ions in the composition becomes 1:1 to 1:1000; the water-soluble polymer be contained in a ratio of 0.1 to 1 part by mass relative to 100 parts by mass of the whole composition; and the organic acid and/or a salt thereof be contained in a ratio of 0.01 to 5 parts by mass relative to 100 parts by mass of the whole composition.

The form of the bactericidal composition for bacterial skin disease of this embodiment obtained by, for example, mixing the above-described respective components is not particularly limited. For example, other than the form of a solution, a mode in which the composition has fluidity, such as a sol and a gel, is conceivable.

The bactericidal composition for bacterial skin disease of this embodiment may further include at least one selected from the group consisting of a surfactant, a humectant, a solvent, and an antioxidant, in an amount of, for example, 100% by mass relative to the composition. These components may be usually added to the composition after the silver salt, the iodide, the water-soluble polymer, and the organic acid and/or a salt thereof are mixed in the solvent to generate the silver iodide nanoparticles. Without being limited to this, for example, the components may be added before the silver iodide nanoparticles are generated.

Examples of the surfactant to be appropriately usedmay include an ionic surfactant and a nonionic surfactant. From the viewpoint of irritation, a nonionic surfactant is preferable. Examples of the nonionic surfactant may include polyoxyalkyl ether, glycerin fatty acid ester, organic acid monoglyceride, polyglycerol fatty acid ester, propylene glycol fatty acid ester, polyglycerol condensed ricinoleic acid ester, sorbitan fatty acid ester, sucrose fatty acid ester, polyoxyethylene fatty acid ester, polyoxyethylene glyceryl isostearate, pyroglutamic acid POE polyhydric alcohol ether ester, polyoxyethylene cetyl ether, and polyoxyethylene sorbitan fatty acid ester. When the surfactant is formulated, the bactericidal composition for bacterial skin disease of this embodiment can easilybe spread uniformly in the state of fine micelles on even the skin surface of a pet densely covered with hairs such as a dog. Furthermore, the nanoparticles of silver iodide as an active ingredient become further easily permeated into epidermal cells. For this reason, the silver iodide nanoparticles are easy to be brought into contact with causative bacteria.

Examples of the humectant may include various intercorneocyte lipids such as ceramides and phytosphingosine; a hyaluronic acid salt such as sodium hyaluronate, and a derivative of the hyaluronic acid; and various polyols such as polypropylene glycol, polyethylene glycol, ethyl hexanediol, hexylene glycol, glycerin, propylene glycol, sorbitol, hexanediol, and capryl glycol. Among these, ceramides and phytosphingosine are preferable, because they have the excellent function of maintaining moisture to the skin, and fix the skin texture. Especially, ceramides are preferable, since they are a substance that is particularly needed by the damaged skin.

As the solvent other than water, an organic solvent may be added in order to improve wettability or the like. Examples of the organic solvent may include alcohol such as ethanol and isopropanol; glycol ether such as diethylene glycol monomethyl ether, diethylene glycol butyl ether, diethylene glycol monoethyl ether, and diethylene glycol dibutyl ether; polyethylene glycol such as polyethylene glycol-300 and polyethylene glycol-400; glycol such as propylene glycol and glycerin; pyrrolidone such as 2-pyrrolidone and N-methyl-2-pyrrolidone; glycerol formal; dimethyl sulfoxide; dibutyl sebacate; polyoxyethylene sorbitan ester such as polysorbate 80; and a mixture thereof.

Furthermore, in order to prevent deterioration of the bactericidal composition for bacterial skin disease of this embodiment, an antioxidant may be suitably used. Specific examples of the antioxidant to be appropriately selected may include sodium bisulfite, sodium sulfite, sodium metabisulphite, sodium thiosulfate, sodium formaldehyde sulfoxylate, 1-ascorbic acid, erythorbic acid, acetylcysteine, cysteine, monothioglycerol, thioglycolic acid, thiolactic acid, thiourea, dithiothreitol, dithioerythreitol, glutathione, ascorbyl palmitate, butylated hydroxyanisole, butylated hydroxytoluene, nordihydroguaiaretic acid, propyl gallate, α-tocopherol, and a mixture thereof.

The bactericidal composition for bacterial skin disease obtained as described above can be used in various ways. For example, by periodically coating the skin of a pet having developed a bacterial skin disease such as pyoderma or a pet not having developed a bacterial skin disease with the bactericidal composition for bacterial skin disease of this embodiment after a usual shampoo, the bacteria on the skin can be killed, and bacterial skin diseases such as pyoderma can be treated or prevented.

The causative bacteria of bacterial skin diseases including pyoderma in a pet is, for example, bacteria normally present in the skin. Although bacteria normally exist in the skin, a certain factor (such as stresses and underlying diseases) on the living body side causes proliferation of bacteria, leading to occurrence of an inflammatory reaction. Thus, bacterial skin diseases are developed. Examples of the bacteria normally present in the skin may include Staphylococcus pseudintermedius, Staphylococcus schleiferi, Staphylococcus aureus, Staphylococcus hyicus, Pseudomonas aeruginosa, Proteus mirabilis, Klebsiella, Escherichia coli, Enterobacter, Actinomyces, Nocardia, and Mycobacterium.

As described above, according to this embodiment, the silver iodide as an inorganic compound, for example, is used as one of active ingredients instead of using an antibiotic like in the conventional case. Therefore, it is difficult for bacteria to acquire drug resistance. Furthermore, since nanoparticles are used, the nanoparticles remain in the affected area for an extended period after coating, and can maintain their effects.

Furthermore, in the bactericidal composition for bacterial skin disease of this embodiment, the iodide ions are adsorbed by the silver iodide nanoparticles to thereby form the electric double layer around the silver iodide nanoparticles. The silver iodide nanoparticles, around which such an electric double layers are formed, are protected by the water-soluble polymer. Furthermore, the organic acid and/or a salt thereof contained in the bactericidal composition for bacterial skin disease of this embodiment suppress aggregation of the silver iodide nanoparticles. Therefore, according to this embodiment, there can be obtained the bactericidal composition for bacterial skin disease including the silver iodide nanoparticles that have excellent dispersibility. Since the silver iodide particles are dispersed in the form of nanoparticles, the bactericidal effects can be effectively obtained even in a small amount.

Furthermore, the bactericidal composition for bacterial skin disease of this embodiment can be provided, for example, in the form of having fluidity, and can be applied to products such as a spray and a lotion.

In addition, when the bactericidal composition for bacterial skin disease of this embodiment includes an oxycarboxylic acid such as citric acid as the organic acid and/or salt, reaggregation of the silver iodide nanoparticles can be further suppressed. Therefore, the performance can be maintained for a longer period.

Next, thepresent invention will be describedmore specifically by illustrating examples. However, the present invention is not limited to only these examples.

### Examples

### (Example 1)

To 320 mL of a 0.8 M potassium iodide solution (purity KI 99% (manufactured by Wako Pure Chemical Industries, Ltd.)), 1 wt% of polyvinylpyrrolidone (manufactured by Across organics, molecular weight 3500), and 0.02 wt% of citric acid (purity 98% (manufactured by Wako Pure Chemical Industries, Ltd.) ) were added, and the mixture was stirred until it was completely dissolved. In another container, 50 mL of a 0. 5M silver nitrate solution (purity 99.8% (manufactured by Wako Pure Chemical Industries, Ltd.)) was prepared within a shielding container. The above-described two solutions were instantaneously stirred and mixed to thereby obtain a bactericidal composition for bacterial skin disease of Example 1. The mixing ratio of the above-described two solutions was set 1:2 in terms of the molar ratio of silver ions : iodide ions when mixed, such that the molar ratio of silver contained in the silver iodide nanoparticles in the composition and iodide ions existing as ions in the composition becomes 1:1. Here, the particle size of the silver iodide nanoparticles contained in the obtained bactericidal composition for bacterial skin disease was measured by a zeta potential and particle size measuring system (manufactured by Otsuka Electronics Co. , Ltd. , laser Dopple-rmethod (dynamic electrophoretic light scattering method) ). The average particle size at this time was 80.0 nm. It is noted that, as described herein, the average particle size indicates the volume average particle size.

### (Example 2)

A bactericidal composition for bacterial skin disease was obtained in the same method as that in Example 1 except that the mixing ratio was set such that the molar ratio of silver ions : iodide ions becomes 1:5 (the molar ratio (theoretical value) of silver contained in the silver iodide nanoparticles of the obtained composition and iodide ions existing as ions in the obtained composition is 1:4). The average particle size of the contained silver iodide nanoparticles was 27.3 nm.

### (Example 3)

A bactericidal composition for bacterial skin disease was obtained in the same method as that in Example 1 except that the mixing ratio was set such that the molar ratio of silver ions : iodide ions becomes 1:10 (the molar ratio (theoretical value) of silver contained in the silver iodide nanoparticles of the obtained composition and iodide ions existing as ions in the obtained composition is 1:9). The average particle size of the contained silver iodide nanoparticles was 4.2 nm. Furthermore, this silver iodide nanoparticle composition of Example 3 was observed by a transmission electron microscope (manufactured by JEOL Ltd., JEM-2100). The photograph is shown in FIG. 1.

### (Example 4)

1.0 wt% of the bactericidal composition for bacterial skin disease obtained in Example 3, 0.5 wt% of potassium iodide (purity KI 99% (manufactured by Wako Pure Chemical Industries, Ltd.) ), 1.0 wt% of citric acid monohydrate (purity 98% (manufactured by Wako Pure Chemical Industries, Ltd.)), 0.1 wt% of polyvinylpyrrolidone (manufactured by Across organics, molecular weight 3500), and a remainder of pure water were mixed to prepare a composition having a ratio of the silver iodide lower than those in the compositions of Examples 1 to 3. The molar ratio of silver ions : iodide ions of this composition (silver ions when mixing the solution of Example 3 : sum of iodide ions when mixing the solution of Example 3 and iodide ions derived from potassium iodide newly added in Example 4) was 1:56. The molar ratio (theoretical value) of silver contained in the silver iodide nanoparticles of the obtained composition and iodide ions existing as ions in the obtained composition is 1:55. The average particle size of the contained silver iodide nanoparticles was 3.8 nm.

### (Example 5)

1.0 wt% of the bactericidal composition for bacterial skin disease obtained in Example 3, 1.0 wt% of potassium iodide (purity KI 99% (manufactured by Wako Pure Chemical Industries, Ltd. ) ), 0.5 wt% of citric acid monohydrate (purity 98% (manufactured by Wako Pure Chemical Industries, Ltd.)), 0.1 wt% of polyvinylpyrrolidone (manufactured by Across organics, molecular weight 3500), and a remainder of pure water were mixed to prepare a composition having a ratio of the silver iodide lower than those in the compositions of Examples 1 to 4. The molar ratio of silver ions : iodide ions of this composition (silver ions when mixing the solution of Example 3: sum of iodide ions when mixing the solution of Example 3 and iodide ions derived from potassium iodide newly added in Example 5) was 1:100. The molar ratio (theoretical value) of silver contained in the silver iodide nanoparticles of the obtained composition and iodide ions existing as ions in the obtained composition is 1:99. The average particle size of the contained silver iodide nanoparticles was 2.5 nm.

### (Example 6)

0.3 wt% of the bactericidal composition for bacterial skin disease obtained in Example 3, 3.0 wt% of potassium iodide (purity KI 99% (manufactured by Wako Pure Chemical Industries, Ltd. ) ), 0.1 wt% of citric acid monohydrate (purity 98% (manufactured by Wako Pure Chemical Industries, Ltd.)), and a remainder of pure water were mixed to prepare a composition having a ratio of the silver iodide lower than those in the compositions of Examples 1 to 5. The molar ratio of silver ions : iodide ions of this composition (silver ions when mixing the solution of Example 3:sum of iodide ions when mixing the solution of Example 3 and iodide ions derived from potassium iodide newly added in Example 6) was 1 : 800. The molar ratio (theoretical value) of silver contained in the silver iodide nanoparticles of the obtained composition and iodide ions existing as ions in the obtained composition is 1:799. The average particle size of the contained silver iodide nanoparticles was 1.3 nm.

### (Comparative Example 1)

0.5 wt% of potassium iodide (purity KI 99% (manufactured by Wako Pure Chemical Industries, Ltd.)), 1.0 wt% of citric acid monohydrate (purity 98% (manufactured by Wako Pure Chemical Industries, Ltd.)), 0.1 wt% of polyvinylpyrrolidone (manufactured by Across organics, molecular weight 3500), and a remainder of pure water were mixed to prepare a solution containing no silver iodide.

### (Comparative Example 2)

A potassium iodide solution was prepared in the same manner as that in Example 1, and a 5 M silver nitrate solution was prepared in another container within a shielding container. The above-described solutions were stirred and mixed such that the molar ratio of silver ions : iodide ions becomes 1:1, to obtain a silver iodide nanoparticle composition. The average particle size at this time was 180.8 nm.

### (Comparative Example 3)

A potassium iodide solution was prepared in the same manner as that in Example 1, and a 10 M silver nitrate solution was prepared in another container within a shielding container. The above-described solutions were stirred and mixed such that the molar ratio of silver ions : iodide ions becomes 2:1, to obtain a silver iodide nanoparticle composition. The average particle size at this time was 626.9 nm.

Furthermore, after the synthesis of the obtained silver iodide nanoparticle composition, the particles were immediately deposited.

### (Comparative Example 4)

0.1 wt% of the bactericidal composition for bacterial skin disease obtained in Example 3, 1.4 wt% of potassium iodide (purity KI 99% (manufactured by Wako Pure Chemical Industries, Ltd.)), 0.1 wt% of citric acid monohydrate (purity 98% (manufactured by Wako Pure Chemical Industries, Ltd.)), and a remainder of pure water were mixed to prepare a composition having a ratio of the silver iodide lower than those in the compositions of Examples. The molar ratio of silver ions : iodide ions of this composition (silver ions when mixing the solution of Example 3: sum of iodide ions when mixing the solution of Example 3 and iodide ions derived from potassium iodide newly added in Comparative Example 4) was 1:1250. Existence of silver iodide nanoparticles could not be confirmed.

### (Bactericidal Test Method)

The composition of each of Examples and Comparative Examples in an amount of 0.1 mL, and a bacterial suspension of coliformbacteria (Escherichia coli), Staphylococcus aureus, or a Staphylococcus pseudintermedius meticillin resistant strain isolated from a pyoderma case dog each in an amount of 0.1 mL were mixed, to obtain a test sample. Each sample was reacted at room temperature for 5 minutes while being stirred using a microtube rotator. After the stirring for 5 minutes, 1 mL of an SCDLP culture medium was added in order to stop the reaction of the bacteria with the compound derived from each composition. After that, each sample was diluted with an SCDLP culture medium to 10⁻² to 10⁻⁵ (ten serial dilutions), and 1 mL of the diluted sample was applied to a petri dish and incorporated with a dissolved NB agar culture medium. Then, cultivation was performed at 37°C. The number of formed colonies (CFU/1 mL, Log 10) ( (CFU: colony-forming unit) ) was calculated thereby to evaluate bactericidal properties to each bacterium for each composition. The results are indicated in Table 1.

**[Table 1]**

| | MOLAR RATIO IN MATERIAL (Ag: I) | AVERAGE PARTICLE SIZE (nm) | COUNT OF VIRAL MATERIAL (CFU/mL Log10) | | |
|---|---|---|---|---|---|
| | | | ESCHERICHIA COLI | STAPHYLOCOCCUS AUREUS | RESISTANT BACTERIA |
| EXAMPLE 1 | 1 : 2 | 80.0 | <1.78 | <1.78 | < 1.78 |
| EXAMPLE 2 | 1 : 5 | 27.3 | <1.78 | <1.78 | <1.78 |
| EXAMPLE 3 | 1 : 10 | 4.2 | <1.78 | <1.78 | <1.78 |
| EXAMPLE 4 | 1 : 56 | 3.8 | <1.78 | <1.78 | <1.78 |
| EXAMPLE 5 | 1 : 100 | 2.5 | <1.78 | <1.78 | <1.78 |
| EXAMPLE 6 | 1 : 800 | 1.3 | 2.08 | 2.26 | 2.62 |
| COMPARATIVE EXAMPLE 1 | - | - | 5.08 | 5.64 | 5.82 |
| COMPARATIVE EXAMPLE 2 | 1 : 1 | 180.8 | 3.78 | 4.51 | 3.91 |
| COMPARATIVE EXAMPLE 3 | 2 : 1 | 626.8 | 5.52 | 5.91 | 6.30 |
| COMPARATIVE EXAMPLE 4 | 1 : 1250 | - | - | - | - |
| CONTROL | - | - | 6.83 | 6.68 | 6.78 |

From the above results, it was confirmed that the bactericidal compositions for bacterial skin diseases of Examples 1 to 5 show high bactericidal properties of the detection limit or less in any bacterium. In addition, it was confirmed that although Example 6 does not have a value equal to or lower than the detection limit, the composition still shows high bactericidal properties of 99.99%. On the other hand, in Comparative Example 1 in which the silver iodide as an active ingredient was not contained and Comparative Example 3 in which the silver iodide nanoparticles were deposited, bactericidal effects were hardly exerted. In Comparative Example 2, bactericidal effects were also low. From these results, it was confirmed that the bactericidal composition for bacterial skin disease of this embodiment has high effects on not only normally existing bacteria but also antibiotic resistant bacteria.

### (Example 7)

1.0 wt% of the bactericidal composition for bacterial skin disease obtained in Example 3, 0.5 wt% of potassium iodide (purity KI 99% (manufactured by Wako Pure Chemical Industries, Ltd. ) ), 1.0 wt% of citric acid monohydrate (purity 98% (manufactured by Wako Pure Chemical Industries, Ltd.)), 0.1 wt% of polyvinylpyrrolidone (manufactured by Across organics, molecular weight 3500), 0.01 wt% of a surfactant (EMALEX GWIS-120 manufactured by Nihon Emulsion Co., Ltd.), and a remainder of pure water were mixed to prepare a composition having the same molar ratio of the silver ions : iodide ions as that of the composition of Example 4. The molar ratio of silver ions : iodide ions of this composition (silver ions when mixing the solution of Example 3:sum of iodide ions when mixing the solution of Example 3 and iodide ions derived from potassium iodide newly added in Example 7) was 1:56. The molar ratio (theoretical value) of silver contained in the silver iodide nanoparticles of the obtained composition and iodide ions existing as ions in the obtained composition is 1:55. The average particle size of the contained silver iodide nanoparticles was 3.8 nm.

### (Example 8)

1.0 wt% of the bactericidal composition for bacterial skin disease obtained in Example 3, 1.0 wt% of potassium iodide (purity KI 99% (manufactured by Wako Pure Chemical Industries, Ltd. ) ), 0.5 wt% of citric acid monohydrate (purity 98% (manufactured by Wako Pure Chemical Industries, Ltd.)), 0.1 wt% of polyvinylpyrrolidone (manufactured by Across organics, molecular weight 3500 ), 0.01 wt% of a surfactant (EMALEX GWIS-160 manufactured by Nihon Emulsion Co., Ltd.), and a remainder of pure water were mixed to prepare a composition having the same molar ratio of the silver ions : iodide ions as that of the composition of Example 5. The molar ratio of silver ions : iodide ions of this composition (silver ions when mixing the solution of Example 3:sum of iodide ions when mixing the solution of Example 3 and iodide ions derived from potassium iodide newly added in Example 8) was 1:100. The molar ratio (theoretical value) of silver contained in the silver iodide nanoparticles of the obtained composition and iodide ions existing as ions in the obtained composition is 1:99. The average particle size of the contained silver iodide nanoparticles was 2.5 nm.

### (Example 9)

0.3 wt% of the bactericidal composition for bacterial skin disease obtained in Example 3, 3.0 wt% of potassium iodide (purity KI 99% (manufactured by Wako Pure Chemical Industries, Ltd. ) ), 0.1 wt% of citric acid monohydrate (purity 98% (manufactured by Wako Pure Chemical Industries, Ltd. ) ), 0.01 wt% of a surfactant (PYROTER CPI60 manufactured by Nihon Emulsion Co., Ltd.), and a remainder of pure water were mixed to prepare a composition having the same molar ratio of the silver ions : iodide ions as that of the composition of Example 6. The molar ratio of silver ions : iodide ions of this composition (silver ions when mixing the solution of Example 3: sum of iodide ions when mixing the solution of Example 3 and iodide ions derived from potassium iodide newly added in Example 9) was 1:800. The molar ratio (theoretical value) of silver contained in the silver iodide nanoparticles of the obtained composition and iodide ions existing as ions in the obtained composition is 1:799. The average particle size of the contained silver iodide nanoparticles was 1.3 nm.

### (Bactericidal Test Method Using Animal Skin)

Next, a bactericidal test using a real animal skin was performed. As the animal skin, the fur of a rabbit was used.

The fur of a rabbit was cut into a diameter of about 3 cm, and the cut fur was placed in a plastic petri dish, on which a bacterial suspension (10^{7.19} bacteria/mL) of a Staphylococcus pseudintermedius meticillin resistant strain was dropped in an amount of 0. 5 mL each. The fur of a rabbit was left to stand for one hour thereby to dry the bacterial solution. Thus, an animal skin to which bacteria was attached was prepared. The composition of each example was poured in a spray container having a discharge amount of 0.15 ml, sprayed on the animal skin attached with bacteria three times, and left to stand in a constant temperature bath at 37°C for 6 hours. After 6 hours, 10 mL of an SCDLP culture medium was added to wash out the bacteria. After that, each sample was diluted with an SCDLP culture medium to 10⁻² to 10⁻⁵ (ten serial dilutions), and 1 mL of the diluted sample was placed in a petri dish and incorporated with a dissolved NB agar culture medium. Then, cultivation was performed at 37°C. The number of formed colonies (Log CFU) ((CFU: colony-forming unit)) was calculated to evaluate bactericidal properties of each composition on the animal skin. The results are indicated in Table 2. It is noted that control is the number of bacteria present on the animal skin after the animal skin attached with bacteria was left to stand in an incubator at 37°C for 6 hours without spraying.

**[Table 2]**

| | MOLAR RATIO IN MATERIAL (Ag : I) | AVERAGE PARTICLE SIZE (nm) | COUNT OF VIAL BACTERIA (RESISTANT BACTERIA) (log CFU) |
|---|---|---|---|
| EXAMPLE 4 | 1 : 56 | 3.8 | 1.93 |
| EXAMPLE 5 | 1 : 100 | 2.5 | 2.17 |
| EXAMPLE 6 | 1 : 800 | 1.3 | 2.81 |
| EXAMPLE 7 | 1 : 56 | 3.8 | < 1.00 |
| EXAMPLE 8 | 1 : 100 | 2.5 | < 1.00 |
| EXAMPLE 9 | 1 : 800 | 1.3 | 1.08 |
| CONTROL | - | - | 4.07 |

From the above results, it was confirmed that in the bactericidal test using a real animal skin, the compositions of Examples 4 to 6 have bactericidal properties of 99.996% to 99.999%, while the compositions containing a surfactant of Examples 7 to 9 have bactericidal properties of the detection limit or less, or have higher bactericidal properties of 99.9998%. It is considered that since the real animal skin is covered with a sebum component, the addition of a surfactant caused permeability into the skin to be further enhanced. From these results, it was confirmed that the bactericidal composition for bacterial skin disease of this embodiment also shows high bactericidal properties on the real animal skin.

## Claims

1. A bactericidal composition for bacterial skin disease, comprising:
silver iodide in a form of nanoparticles having a particle size of 1 nm or more and 100 nm or less;
iodide ions;
at least one type of water-soluble polymer; and
an organic acid having a carboxyl group and/or a salt thereof,
wherein
a molar ratio of silver in the silver iodide and the iodide ions is 1:1 to 1:1000.

2. The bactericidal composition for bacterial skin disease according to claim 1, further comprising at least one selected from the group consisting of a surfactant, a humectant, a solvent, and an antioxidant.

3. A bactericidal composition for bacterial skin disease, manufactured by:
setting a molar ratio of silver ions constituting a silver salt and iodide ions constituting an iodide such that silver iodide in a form of nanoparticles having a particle size of 1 nm or more and 100 nm or less is contained in the obtained composition, and the molar ratio of silver contained in the silver iodide and iodide ions existing as ions becomes 1:1 to 1:1000; and
mixing, in a solvent, the silver salt and the iodide in which the molar ratio of the silver ions and the iodide ions is set, a water-soluble polymer, and an organic acid having a carboxyl group and/or a salt thereof.

4. A manufacturing method of a bactericidal composition for bacterial skin disease, comprising:
setting a molar ratio of silver ions constituting a silver salt and iodide ions constituting an iodide such that silver iodide in a form of nanoparticles having a particle size of 1 nm or more and 100 nm or less is contained in the obtained composition, and a molar ratio of silver contained in the silver iodide and iodide ions existing as ions is 1:1 to 1:1000; and
mixing, in a solvent, the silver salt and the iodide in which the molar ratio of the silver ions and the iodine ions is set, a water-soluble polymer, and an organic acid having a carboxyl group and/or a salt thereof.
